# EUROPÄISCHE PATENTSCHRIFT

(11) **EP 1 337 299 B1**
(45) Veröffentlichungstag und Bekanntmachung des Hinweises auf die Patenterteilung: **06.06.2007**
(21) Anmeldenummer: 02767073.6
(22) Anmeldetag: 29.07.2002
(51) Int. Cl.: A62B 18/08

(54) **BÄNDERUNG FÜR EINE ATEMSCHUTZMASKE**
HEAD HARNESS FOR A RESPIRATOR
ENSEMBLE DE BRIDES POUR MASQUE DE PROTECTION RESPIRATOIRE

(30) Priorität: 17.08.2001 DE 10140575
(43) Veröffentlichungstag der Anmeldung: 27.08.2003
(73) Patentinhaber: MSA Auer GmbH, 12059 Berlin (DE)
(72) Erfinder: HORN, Michael, 10587 Berlin (DE)
(74) Vertreter: Wablat, Wolfgang
(86) Internationale Anmeldenummer: PCT/DE2002/002862
(87) Internationale Veröffentlichungsnummer: WO 2003/018136

(56) Entgegenhaltungen:
- DE-A- 3 122 034
- DE-U- 29 719 440
- US-A- 5 481 763
- US-A- 5 895 537

## Beschreibung

Die Erfindung betrifft eine Bänderung für eine Atemschutzmaske, bestehend aus einer Kopfplatte und von dieser ausgehenden Stirn- Schläfen- und Nackenbändern, die über eine Sechs-Punkt-Anbindung mit Klemmschnallen an der Atemschutzmaske befestigbar sind.

Eine ähnliche Bänderung mit einer 5-Punkt-Befestigung, bei der zum Halten der Atemschutzmaske am Kopf des Benutzers von einer festen, nicht dehnbaren Kopfplatte ein im Stirnbereich liegendes elastisches Band und zwei elastische Schläfenbänder sowie zwei im Nackenbereich liegende feste Bänder ausgehen, ist beispielsweise aus der EP 046 43 42 bekannt. Die festen Nackenbänder sind über einstellbare Schnallen an den Maskenkörper angeschlossen, um die Bänderung an die Kopfgröße des Benutzers anpassen zu können. Der Tragekomfort einer solchen Maske ist insofern unzureichend, als sich die Bänderung während der Benutzung der Atemschutzmaske lockern kann und ständig nachgestellt werden muß, um eine sichere Fixierung der Maske zu erreichen. Das heißt, gerade unter Belastung ist ein fester Sitz der Maske am Kopf nicht gewährleistet, zumal diese aufgrund des elastischen Stirnbandes unter Last leicht verrutschen kann. Andererseits wird aber durch ein zu straffes Anziehen der Bänderung die Bewegungsfreiheit des Kopfes eingeschränkt. Die nicht elastische Kopfplatte kann beim Anlegen verrutschen.

Bei einer aus der EP 0350 322 bekannten Atemschutzmaske ist zu deren Halterung am Kopf eine biaxial streckbare, aus mehreren Teilen zusammengesetzte und entsprechend der Kopfform gewölbte Haube vorgesehen, die überwiegend unmittelbar mit dem Maskenkörper verbunden ist. Diese Halterung, die den Kopf haubenartig einfaßt, kann sich aufgrund ihrer Elastizität zwar weitestgehend der Kopfform anpassen, ein sicherer Halt der mit einem Atemschutzfilter belasteten Maske ist jedoch nicht gewährleistet, d.h., durch das Verrutschen während des Einsatzes ist der Tragekomfort beeinträchtigt.

Weitere Ausführungsformen der Bänderungen sind aus DE 29719440, US-A-5895537, DE-A-3122034 oder US-A-5481763 bekannt.

Der Erfindung liegt die Aufgabe zugrunde, eine Bänderung der eingangs erwähnten Art für eine Atemschutzmaske anzugeben, die bei unterschiedlicher Kopfgröße eine gute Paßform der Bänderung und bei freier Beweglichkeit des Kopfes einen festen Sitz der Maske sowie deren bequemes und schnelles Anlegen gewährleistet.

Erfindungsgemäß wird die Aufgabe mit einer gemäß den Merkmalen des Patentanspruchs 1 ausgebildeten Bänderung gelöst.

Aus den Unteransprüchen ergeben sich weitere Merkmale und vorteilhafte Weiterbildungen der Erfindung.

Ausgehend von den bekannten Bänderungen mit Sechs-Punkt-Anbindung am Maskenkörper, bei denen von einer Kopfplatte jeweils an die Maske anschließbare Stirn-, Schläfen- und Nackenbänder ausgehen, besteht der wesentliche Erfindungsgedanken darin, daß die Kopfplatte aus einem ausschließlich in Querrichtung (X-Richtung) elastisch dehnbaren Gestrick besteht, das in Längsrichtung (Y-Richtung) nicht dehnbar ist. Auch die in Querrichtung verlaufenden Schläfen- und Nackenbänder sind in dieser Richtung dehnbar. Hingegen ist das in Längsrichtung verlaufende Stirnband nicht elastisch.

Eine derart ausgebildete Bänderung ist in der Lage, unterschiedliche Kopfgrößen auszugleichen und sich verschiedenen Kopfformen anzupassen und in jedem Fall einen festen, sicheren Sitz und einen hohen Tragekomfort zu gewährleisten. Die in Querrichtung wirkenden Kräfte werden über die in dieser Richtung elastische Kopfplatte gleichmäßig verteilt, während die am Maskenkörper wirkenden Längskräfte über die festen Stirnbänder übertragen und von der Kopfplatte aufgenommen werden. Aufgrund der elastischen Kräfteverteilung über die Kopfplatte und die Schläfen- und Nackenbänder liegt die Maske mit gleichmäßigem Druck und dicht am Gesicht des Benutzers an und die auftretenden Längskräfte können aufgenommen werden. Trotz fest anliegender Atemschutzmaske ist die Beweglichkeit des Kopfes nicht eingeschränkt.

In weiterer Ausbildung der Erfindung sind die von der Kopfplatte ausgehenden Stirn- und Schläfenbänder entsprechend der jeweiligen Kopfgröße und -form fest voreinstellbar, während nur die Nackenbänder nach dem Anlegen der Atemschutzmaske nachgezogen werden. Dadurch ist die Atemschutzmaske nach dem Anlegen sofort und von selbst lagerichtig positioniert und vorfixiert. Anschließend werden die Nackenbänder so weit gespannt, daß die Atemschutzmaske nebst Bänderung fest und komfortabel am Kopf sitzt.

Ein weiteres wichtiges Erfindungsmerkmal besteht in der Ausbildung der Stirn-, Schläfen- und Nackenbänder jeweils aus nur einem einzigen, durchgehenden Band. Dadurch wird zum einen die Kraftverteilung in Querrichtung und die Kraftübertragung in Längsrichtung vergleichmäßigt und zum anderen die Lebensdauer der Bänderung erhöht.

In Ausgestaltung der Erfindung ist die Kopfplatte aus einem unter dem Handelsnamen NOMEX^{®} bekannten hitze- und chemikalienbeständigem Gestrick mit den oben angegebenen Dehnungseigenschaften gefertigt und als Doppeltrapez ausgebildet, an dessen oberem Rand das Stirnband, an dessen unterem Rand das Nackenband und in Höhe dessen von den beiden Trapezen gebildeter gemeinsamer längerer Grundlinie, d.h. an der breitesten Stelle, das Schläfenband verläuft.

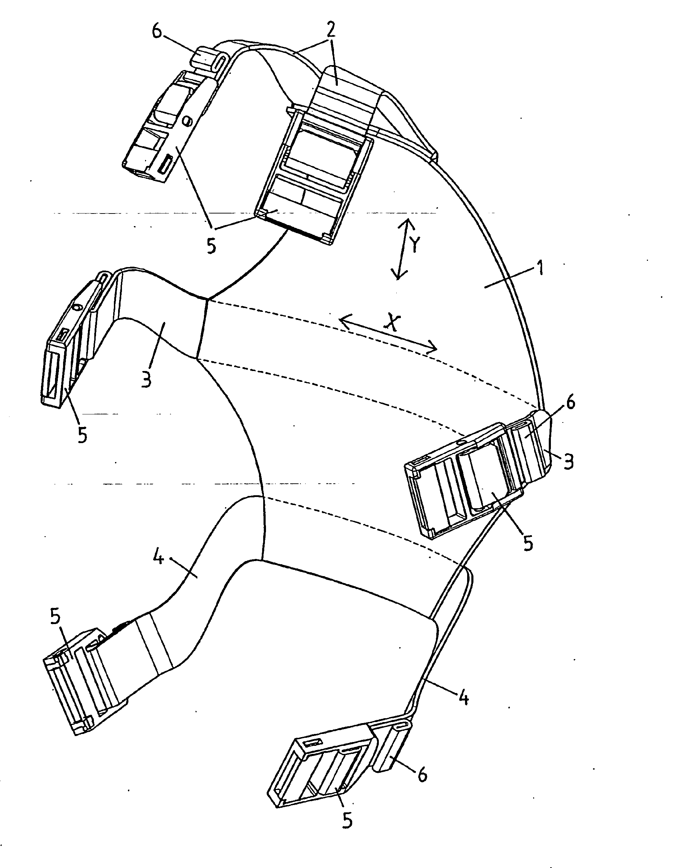

Ein Ausführungsbeispiel der Erfindung wird anhand der Zeichnung näher erläutert. In der einzigen Figur ist in perspektivischer Darstellung eine Bänderung mit Sechs-Punkt-Anbindung am Maskenkörper wiedergegeben,

Die Bänderung besteht aus einer Kopfplatte 1 sowie einem Stirnband 2, einem Schläfenband 3 und einem Nackenband 4. Die Kopfplatte 1 hat die Form eines Doppel-Trapezes, d.h., von zwei an den jeweils langen Grundseiten aneinanderstoßenden Trapezen. Sie besteht aus einem nicht brennbaren, chemisch stabilen und temperaturbeständigen Gestrick, das unter dem Handelsnamen NOMEX^{®} bekannt ist. Ein wesentliches Merkmal der Kopfplatte 1 bzw. des für diese verwendeten Gestricks besteht darin, daß es in Querrichtung hochelastisch ist, aber in Längsrichtung im wesentlichen nicht dehnbar ist. Das Stirnband 2, das Schläfenband 3 und das Nackenband 4 bestehen jeweils aus einem einzigen durchgehenden Band, das mit NOMEX-Gestrick umwebt ist und auf der vom Kopf des Benutzers abgewandten Seite der Kopfplatte angebracht ist. Das Stirnband 2, das an zwei Punkten im Stirnbereich mit dem Maskenkörper (nicht dargestellt) verbunden ist, besteht aus einem nicht dehnbaren Grundmaterial. Es ist vollflächig am oberen Rand der Kopfplatte 1 in Querrichtung vernäht, während seine freien Enden in Längsrichtung der Kopfplatte 1 verlaufen. Dadurch wird in Längsrichtung eine stabile Verbindung zwischen der Kopfplatte 1 und dem Maskenkörper (nicht dargestellt) geschaffen. Das heißt, auch unter dem Gewicht eines mit dem Maskenkörper verbundenen Filters kann die Atemschutzmaske selbst bei den im Einsatzfall auftretenden Belastungen nicht nach unten verrutschen, da in Längsrichtung keine Dehnung auftritt und die Kopfplatte 1 in Querrichtung unter elastischer Vorspannung fixiert ist. Um die elastische Kraftwirkung in Querrichtung auf den Maskenkörper (nicht dargestellt) aufbringen zu können, sind in dieser Richtung nicht nur die Kopfplatte 1, sondern auch das Schläfenband 3 und das Nackenband 4 dehnbar ausgebildet. An den beiden freien Enden des jeweiligen Bandes (Stirnband 2, Schläfenband 3, Nackenband 4) sind mit dem Maskenkörper verbindbare Klemmschnallen 5 bis 10 vorgesehen, wobei mit den Klemmschnallen 5 bis 8 - entsprechend der Kopfgröße und -form des jeweiligen Benutzers - eine feste Voreinstellung der Bandlänge des Stirnbandes 2 und des Schläfenbandes 3 erfolgt. Im oberen Bereich der Bänderung, d.h. in den oberen vier Anbindungspunkten wird somit bereits vorab deren benutzerspezifische exakte Paßform fest eingestellt. Die Klemmschnallen 9 und 10 sind am Nackenband 4 nicht fest voreingestellt, sondern befinden sich in einer eine große Nackenbandlänge erzeugenden Lage, so daß die Maske bequem und schnell angelegt werden kann. Nach dem Anziehen der beiden Nackenbandteile ist die Atemschutzmaske horizontal fest am Kopf des Benutzers verspannt und gleichermaßen in Längsrichtung fixiert. Die Elastizität des Nackenbandes 4 erlaubt dennoch ungehinderte Kopfbewegungen des Benutzers. Die beiden Enden des Stirnbandes 2, des Schläfenbandes 3 und des Nackenbandes 4 wurden nach dem Aufziehen der Klemmschnallen 5 bis 10 zu einer Wulst 6 umgenäht, so daß die Bandenden nicht aus der betreffenden Klemmschnalle herausrutschen können. Mit der durchgehend ausgebildeten Ausführung der Bänder und der an den freien Enden vorgesehenen Wulst 6 sind des weiteren keine offenen Bandenden vorhanden, aus denen die in den elastischen Schläfen- und Nackenbändern 3, 4 liegenden Gummifäden herausgezogen werden könnten.

### Bezugszeichenliste

- 1: Kopfplatte
- 2: Stirnband
- 3: Schläfenband
- 4: Nackenband
- 5: Klemmschnalle
- 6: Wulst

## Patentansprüche

1. Bänderung für eine Atemschutzmaske, bestehend aus einer Kopfplatte und von dieser ausgehenden Stirn-, Schläfen- und Nackenbändern, die über eine Sechs-Punkt-Anbindung mit Klemmschnallen an der Atemschutzmaske befestigbar sind, **dadurch gekennzeichnet, daß** die Kopfplatte (1) aus einem in Querrichtung (x) elastisch dehnbaren und in Längsrichtung (y) im wesentlichen nicht dehnbaren Material besteht und die in Querrichtung (x) von der Kopfplatte (1) ausgehenden Schläfen- und Nackenbänder (3, 4) in dieser Richtung elastisch dehnbar ausgebildet sind, während die vom oberen Rand der Kopfplatte (1) ausgehenden, in Längsrichtung verlaufenden Stirnbänder (2) aus einem festen nicht dehnbaren Material bestehen.

2. Bänderung nach Anspruch 1, **dadurch gekennzeichnet, daß** die Länge der Stirnbänder (2) und der Schläfenbänder (3) entsprechend der Kopfgröße des Benutzers zur lagerichtigen Positionierung und Vorjustierung der Atemschutzmaske nebst Bänderung mittels der Klemmschnallen (5 bis 8) fest voreinstellbar ist, während die Klemmschnallen (9, 10) der Nackenbänder (4) zum Spannen der Nackenbänder (4) und endgültigen Fixieren der Atemschutzmaske vorgesehen sind.

3. Bänderung nach Anspruch 1 und 2, **dadurch gekennzeichnet, daß** das Stirnband (2), das Schläfenband (3) und das Nackenband (4) jeweils aus einem einzigen durchgehenden Band bestehen, das, jeweils in Querrichtung (x) verlaufend, an der vom Kopf abgewandten Seite der Kopfplatte (1) befestigt ist.

4. Bänderung nach einem der Ansprüche 1 bis 3, **dadurch gekennzeichnet, daß** die Enden der Stirn-, Schläfen- und Nackenbänder (4) zu einer Wulst (6) umgeschlagen und stirnseitig geschlossen sind.

5. Bänderung nach einem der Ansprüche 1 bis 4, **dadurch gekennzeichnet, daß** die in Querrichtung dehnbare Kopfplatte (1) aus einem Gestrick besteht.

6. Bänderung nach einem der Ansprüche 1 bis 5, **dadurch gekennzeichnet, daß** die Kopfplatte (1) die Form von zwei an den Grundlinien aneinanderstoßenden Trapezen aufweist, wobei das Stirnband (2) an deren oberem Rand, das Nackenband (4) an deren unterem Rand und das Schläfenband (3) längs der aneinanderstoßenden Grundlinien des Doppeltrapezes verläuft.

## Claims

1. A harness for a respiratory mask consisting of a head plate and front, temple, and neck straps protruding from it that can be fastened with strap buckles to the respiratory mask in a six-point arrangement, **characterized in that** the head plate (1) is made of a material that can be elastically expanded in a crosswise direction (x) but is substantially non-expandable in longitudinal direction (y), and **in that** the temple and neck straps (3, 4) that protrude crosswise (x) from the head plate (1) can also be stretched in this direction while the front straps (2) that emerge from the upper rim of the head plate (1) and run in longitudinal direction consist of a rigid, non-expandable material.

2. The harness according to claim 1, **characterized in that** the length of the front straps (2) and the temple straps (3) can be preset to the user's head size for correct positioning and preadjustment of the respiratory mask and harness using the strap buckles (5 to 8) while the strap buckles (9, 10) of the neck straps (4) are used for tightening the neck straps (4) and for final fastening of the respiratory mask.

3. The harness according to claims 1 and 2, **characterized in that** the front strap (2), the temple strap (3), and the neck strap (4) each consist of a single continuous strap that runs in crosswise direction (x) and is attached to the side of the head plate (1) that faces away from the wearer's head.

4. The harness according to any one of claims 1 through 3, **characterized in that** the ends of the front, temple, and neck straps (4) are flipped over to form a bulge (6) and closed at the front.

5. The harness according to any one of claims 1 through 4, **characterized in that** the head plate (1) that can be stretched in a crosswise direction consists of a knitted fabric.

6. The harness according to any one of claims 1 through 5, **characterized in that** the head plate (1) has the shape of two trapezoids abutting along their base lines while the front strap (2) runs along its upper rim, the neck strap (4) along its lower rim, and the temple strap (3) runs along the abutting base lines of said double trapezoid.

## Revendications

1. Agencement de brides pour un masque de protection respiratoire, constitué par une plaque de tête et par des brides de front, de tempes et de nuque partant de celle-ci, lesquelles peuvent être fixées sur le masque de protection respiratoire par des boucles de serrage via une fixation en six points, **caractérisé en ce que** la plaque de tête (1) est constituée par un matériau extensible élastiquement en direction transversale (x) et sensiblement non extensible élastiquement en direction longitudinale (y), et les brides de tempe et de nuque (3, 4) partant de la plaque de tête (1) en direction transversale (x) sont réalisées élastiquement extensibles dans cette direction, tandis que les brides de front (2) partant du bord supérieur de la plaque de tête (1) et s'étendant en direction longitudinale sont constituées par un matériau ferme non extensible.

2. Agencement de brides selon la revendication 1, **caractérisé en ce que** la longueur des brides de front (2) et des brides de tempe (3) peut être fermement préréglée au moyen des boucles de serrage (5 à 8) selon la taille de la tête de l'utilisateur pour effectuer le positionnement en bonne position et pour ajuster préalablement le masque de protection respiratoire, tandis que les boucles de serrage (9, 10) des brides de nuque (4) sont prévues pour serrer les brides de nuque (4) et pour fixer définitivement le masque de protection respiratoire.

3. Agencement de brides selon la revendication 1 et 2, **caractérisé en ce que** la bride de front (2), la bride de tempe (3) et la bride de nuque (4) sont constituées chacune par une bride unique continue qui, en s'étendant chacune en direction transversale (x), est fixée sur le côté de la plaque de tête (1) détourné de la tête.

4. Agencement de brides selon l'une des revendications 1 à 3, **caractérisé en ce que** les extrémités des brides de front, de tempe et de nuque (4) sont rabattues pour former un bourrelet (6) et fermées côté frontal.

5. Agencement de brides selon l'une des revendications 1 à 4, **caractérisé en ce que** la plaque de tête (1) extensible en direction transversale est constituée par un maillage.

6. Agencement de brides selon l'une des revendications 1 à 5, **caractérisé en ce que** la plaque de tête (1) présente la forme de deux trapèzes contigus sur les lignes de base, la bride de front (2) s'étendant sur leur bord supérieur, la bride de nuque (4) s'étendant sur leur bord inférieur, et la bride de tempe (3) s'étendant le long des lignes de base contiguës du double trapèze.
